# EUROPEAN PATENT APPLICATION

(11) **EP 1 935 405 A1**
(43) Date of publication of application: **25.06.2008**
(21) Application number: 06127085.6
(22) Date of filing: 22.12.2006
(51) Int. Cl.: A61K 9/00, A61K 9/16, A61K 9/20, A61K 9/50

(54) **Orally disintegrating tablets**

(71) Applicant: LEK Pharmaceuticals d.d., 1526 Ljubljana (SI)
(72) Inventor: The designation of the inventor has not yet been filed
(74) Representative: Demsar, Marija

(57) **Abstract**

The present invention relates to an orally disintegrating tablet comprising micropellets of which at least 50% by weight have a diameter less than 500 µm and one or more excipients; wherein the micropellets comprise a pharmaceutically active ingredient and one or more auxiliary ingredients and are coated with a coating composition. A method of making the orally disintegrating tablet is also provided.

## Description

The present invention relates to orally disintegrating tablets comprising micropellets of diameter less than 500 µm. The micropellets comprise a pharmaceutically active ingredient, which is preferably ketoprofen.

Orally disintegrating tablets are solid dosage forms that disintegrate in the oral cavity leaving an easy-to-swallow residue. Many patients find conventional solid tablets difficult to swallow and orally disintegrating tablets help to overcome this problem.

Currently two main types of technologies are used to obtain orally disintegrating tablets. The first mixes the active ingredient with water-soluble diluents and compresses this on a tableting machine at low to medium compression force. This is the more conventional approach. The second approach involves preparing a suspension of the active ingredient and appropriate excipients, which is then dispensed into blister packs and freeze-dried.

OraSolv® technology from the company Cima provides an oral dosage form which comprises microencapsulated ingredients incorporated into a tablet that dissolves in the mouth without the need for water. The OraSolv® tablets, however, normally need up to 60 seconds to dissolve in the mouth, which is somewhat longer than is desirable.

Recently, Zydis® technology has been developed by the company R.P. Scherer. This is based on the second approach described above and provides an oral drug delivery system which dissolves in 3-5 seconds in the mouth. The final product is obtained by sealing the dried product in special peel-off blister packs - such packs are necessary since tablets obtained by both approaches above lack mechanical resistance.

Other researchers have attempted to improve the disintegration time of tablets by incorporating at least one disintegrant and at least one ingredient accelerating the decomposition of the tablet in the mouth. Said ingredient may be selected from a water-soluble amino acid, a water-soluble amino acid derivative and a water-soluble salt of an amino acid, giving a composition which decomposes in the mouth within 1 to 20 seconds.

An additional problem associated with the administration of orally disintegrating tablets is that those containing taste-objectionable or irritating drugs need to be masked in, at least, the oral cavity. Multiple approaches to taste-masking of bitter drugs have been described in the prior art.

Coating agents are typically used to mask the objectionable taste of drugs. The coating agents used are well known and are selected from polymethacrylates, cellulose polymers, in particular ethyl celluloses, hydroxypropyl-methyl celluloses, hydroxypropyl celluloses and cellulose acetate phthalates.

Methacrylate-based coatings may be purchased under the trade name Eudragit®. Many varieties of Eudragit® coatings are available. Eudragit® S100, for instance, is purchased in the form of a powder and is an anionic polymer of methacrylic acid with a carboxylic acid group. The polymer is soluble above pH 7 and is used to coat drugs which need targeted delivery to the ileum. Coating compositions may be pH dependent, like Eudragit® S100, or pH independent.

Pharmaceutically active compounds are typically provided in many different physical forms. Drugs are commonly manufactured as powders comprising crystals of various particle sizes. It is sometimes desirable to increase the particle sizes to aid delivery and control the bioavailability of the drug. Crystals may be combined into larger particles, herein referred to as micropellets, by a variety of methods, including the stepped rimmed flat plan method, emulsion congealing and cross-linking techniques. Extrusion-spheronisation is an alternative technique which produces spherical particles having a smooth uniform surface. Extrusion screens of 0.5-2mm give particle sizes with diameters in this same range.

There remains an unmet need to provide rapidly disintegrating tablets which comprise a pharmaceutically active ingredient which is coated when in the oral cavity, and released at the appropriate stage in the digestive tract. The tablets need to be robust and disintegrate quickly into micropellets which are easy to swallow when in the mouth, without the need for chewing. This is particularly advantageous for those patients who have difficulty chewing, such as children or the elderly.

In accordance with these requirements, we provide in a first aspect of this invention an orally disintegrating tablet comprising micropellets of which at least 50% by weight have a diameter less than 500 µm and one or more excipients; wherein the micropellets comprise a pharmaceutically active ingredient and one or more auxiliary ingredients and are coated with a coating composition. The orally disintegrating tablet in accordance with this invention typically disintegrates in the mouth in less than 60 seconds. Normally the micropellets are substantially spherical.

Many pharmaceutically active ingredients have characteristics (such as poor flow and cohesiveness) which make them unsuitable for coating with a coating composition directly. Incorporating the pharmaceutically active ingredient into a micropellet together with suitable auxiliary ingredients helps to overcome this problem.

The choice of materials to be used for the coating composition, and the coating composition's thickness, may be varied according to the desired pharmacokinetic profile of the pharmaceutically active ingredient used. The coating composition may be pH sensitive or insensitive and thus can be chosen to control the location of release of the pharmaceutically active ingredient. The orally disintegrating tablet according to this invention typically comprises a single unit dosage form of pharmaceutically active ingredient which has the same drug release properties as the individual micropellets.

According to a second aspect of the present invention we further provide a method of making an orally disintegrating tablet comprising
(i) forming micropellets of which at least 50% by weight have a diameter of less than 500 µm from a pharmaceutically active ingredient and one or more auxiliary ingredients;
(ii) coating the micropellets formed in step (i) with a coating composition; and
(iii) compressing the coated micropellets produced in step (ii), together with one or more excipients to form a tablet.

The micropellets in step (i) are preferably formed using an extrusion-spheronisation technique. The one or more auxiliary ingredients are preferably chosen in order to render the mixture suitable for this technique. Normally the micropellets are substantially spherical.

According to a third aspect of the present invention we provide use of an orally disintegrating tablet according to the first aspect of the invention to administer the pharmaceutically active ingredient to a patient.

It will be appreciated that in a population of micropellets there will be a spread of particle diameters. The term micropellets, as used herein, refers to substantially spherical particles wherein at least 50% of the micropellets by weight have a diameter of less than 500 µm. Preferably, at least 75%, more preferably at least 85% and most preferably at least 95% of the micropellets by weight have a diameter less than 500 µm. In a preferred embodiment, at least 50%, more preferably at least 75%, 85% or 95% of the micropellets by weight have a diameter of less than 350 µm. These diameters are required to ensure that an easy-to-swallow residue is produced in the oral cavity when the tablet is ingested.

Preferably, the micropellets have an diameter of no smaller than 50 µm, more preferably no smaller than 100 µm. By this we mean that no more than 10%, preferably no more than 5% of the micropellets by weight have a diameter less than 50 µm or 100 µm.

The diameter of the micropellets may be measured by any suitable means known in the art. For instance, sieve analysis may be used.

It is furthermore important that the size of the micropellets is of a similar order of magnitude to the particle size of the excipients in order to prevent segregation. Approximating the excipient particles to spheres, the diameter of the micropellets is preferably in the range 0.1-20 times the diameter of the excipient particles, most preferably 0.5 - 5 times.

Pharmaceutically active ingredients may include, without limitation, antacids, analgesics, anti-inflammatories, antibiotics, laxatives, anorexics, antiasthmatics, antidiuretics, antiflatulents, antimigraine agents, antispasmodics, sedatives, antihyperactives, tranquilizers, antihistamines, decongestants, betablockers, hormones and combinations thereof. Preferred active substances are analgesics including non-steroidal antiinflammatory drugs, such as diclofenac, ketoprofen, ibuprofen, aspirin or paracetamol or a pharmaceutically acceptable salt thereof, and hormones, e.g., melatonin. Especially preferred is ketoprofen which may be present either as free acid or as a pharmaceutically acceptable salt thereof, e.g., the potassium or sodium salt. Ketoprofen is a particularly irritating drug that needs to be coated to mask its irritating effects in the oral cavity and oesophagus.

Bitter pharmaceutically active ingredients which may be incorporated into the micropellets include clarithromycin, eszopiclone, atorvastatin and methimazole.

The active ingredient in the micropellets is mixed with one or more auxiliary ingredients, which preferably comprise cellulose or cellulose derivatives such as microcrystalline cellulose. Polysaccharides such as carrageenan are also preferred auxiliary ingredients. The choice of auxiliary ingredient is important, since these play a much more significant role during extrusion-spheronisation than during any other pelletisation process. The ingredients facilitate extrusion and the sphericity of the wet pellets; they also impart strength and integrity to the pellets. Microcrystalline cellulose is particularly preferred since it helps to regulate the water content and distribution in granulation. It is generally used as a filler and spheronisation aid. This substance helps to modify the rheological properties of the formulation and imparts plasticity to the pellets. Carrageenan can be used for the same purpose as microcrystalline cellulose. A further suitable auxiliary ingredient is lactose.

The micropellets preferably additionally comprise one or more auxiliary ingredients selected from one or more disintegrants and/or lubricants and/or wetting agents or surfactants. Suitable disintegrants may be selected from crosslinked sodium carboxymethylcellulose, crosslinked polyvinyl pyrrolidone, crosslinked carboxymethyl starch, other starch variants and microcrystalline cellulose, magnesium aluminum silicate and any combination thereof. Preferably, the disintegrant is crosslinked sodium carboxymethylcellulose.

A suitable wetting agent is polysorbate 80.

Suitable surfactants include ionic surfactants, including anionic surfactants such as sodium lauryl sulfate, non ionic surfactants such as different types of poloxamers, esters of sorbitan and fatty acids (such as Span), esters of polyoxyethylenesorbitan and fatty acids (such as Polysorbate 80). Cationic surfactants or amphiphilic surfactants, such as natural or synthesised lecithins may also be used.

Lubricants are primarily used to avoid sticking of the dried product to the surface of the mould. Examples of lubricants which can be used are magnesium stearate or calcium stearate, stearic acid, polyethyleneglycols, sodium stearyl fumarate, hydrogenated vegetable oil, and behenic acid derivatives. Magnesium stearate and calcium stearate are particularly preferred.

The following table summarises the preferred ingredients which comprise the micropellet, typical percentage by weight relative to the pharmaceutically active ingredient and the preferred percentage by weight:

**Table 1**

| | **% relative to active ingredient** | **preferable % relative to active ingredient** | **% in our examples relative to ketoprofen** |
|---|---|---|---|
| MCC (microcrystalline cellulose) | 5-90 | 10-15 | 12.5 |
| Carrageenan | 5-50 | 5-10 | 6.25 |
| Lactose | 5-50 | 5-10 | 6.25 |
| Disintegrants | | 5-10 | - |
| Lubricants | | 0.1-10 | - |
| Wetting agents or surfactants (NaLS or Tween is most preferable) | | 0.1-10 | 2 |

The micropellets of the present invention preferably have a high pharmaceutically active ingredient content. Typically, the pharmaceutically active ingredient comprises 50-90 % by weight of the micropellets with respect to the total mass of the micropellets. Preferably, the content of the pharmaceutically active ingredient in the micropellet is at least 70% by weight. It is preferred that a small mass of auxiliary ingredients is used in order to give a high concentration of pharmaceutically active ingredient and an appropriate mass of orally disintegrating tablet.

The pharmaceutically active ingredient may have an objectionable taste or be irritating to the gastrointestinal tract. The coating composition should be selected accordingly. The coating composition may be pH dependent or pH independent. In the case of taste masking it is desirable to achieve the taste masking effect with the pH dependent protective coating. Typically, such a coating dissolves below pH 5 and remains intact in the pH range 5-14. As a result, in the oral cavity, which is normally a neutral environment with a pH around 7, the coating protects the pharmaceutically active ingredient and prevents the patient from being exposed to a bitter taste or irritating effect. In the acidic environment of the stomach the coating dissolves and the pharmaceutically active ingredient is released. As a rule, very thin coatings are necessary to protect the pharmaceutically active ingredient when a pH dependent coating is used.

The pH dependent coating of the present invention may be selected from the group consisting of cationic polymers, more preferably polymethacrylates such as aminoalkyl methacrylate copolymers. The polymers marketed under the EUDRAGIT trade mark are particularly preferred.

In the case of pH independent coatings, generally a thicker coating is necessary to protect the pharmaceutically active ingredient. pH independent coatings are typically used in patients with physiologically and/or pathologically elevated gastric pH. In such patients pH dependent coatings do not dissolve in the stomach. As a result, the pharmaceutically active ingredient is not absorbed from the gastro-intestinal tract and the pharmaceutically active ingredient's effect is lost.

The pH independent coating used in the present invention may be selected from the group consisting of ethyl cellulose, hydroxypropyl cellulose, hydroxyethyl cellulose, hydroxypropyl methyl cellulose, carboxymethylcellulose sodium, polyvinyl alcohol, polyvinyl pyrrolidone and any combinations thereof.

Further ingredients may be added to the coating composition to improve its characteristics. Protective film forming polymers can be mixed and processed together with other ingredients, such as polyethylene glycol, lactose, HPMC, HPC, microcrystalline cellulose or other water soluble or hydrophillic substances, to enhance permeability if required.

Other ingredients, such as plasticisers, glidants, pigments, solubilising agents may also be added to the coating composition to improve the suitability of the polymer dispersion for the coating. Micronised talc may be used as a glidant. Aromas may also be added to improve the taste masking effect of the coating composition.

The coating composition typically has a thickness of 2-30 µm, preferably 5-20 µm.

The orally disintegrating tablet of the first aspect of this invention comprises coated micropellets and one or more excipients. The pharmaceutically acceptable excipients preferably comprise one or more fillers, one or more disintegrants, one or more lubricants and glidants, and/or other components for solid dosage forms selected from colourants, flavours and sweeteners.

The orally disintegrating tablet is presented, e.g., as a tablet of a size and shape adapted for direct oral administration to a patient. The tablet is pleasant to take and, once placed into the mouth, will disintegrate substantially and rapidly without any voluntary action by the patient, e.g., chewing. Preferably, the tablet disintegrates within 60 seconds of placing into the mouth, and most preferably within 30 seconds. The tablet is therefore convenient to use for the consumer without the need of water or additional devices.

The mass of the orally disintegrating tablet according to this invention is typically no more than 1000mg, preferably no more than 500mg and most preferably in the range 200-500mg.

The filler of the orally disintegrating tablet may be selected from the group consisting of microcrystalline cellulose (MCC), modified forms of microcrystalline cellulose, lactose, sugars, different types of starch, modified forms of starch, mannitol, sorbitol and other polyols, dextrin, dextran and maltodextrin, calcium carbonate, calcium phosphate and/or hydrogen phosphate, sulphate and any combinations thereof. Fillers are typically used in concentrations of 10-90% of the total weight of the orally disintegrating tablet.

Quick dissolve delivery systems for tablets are particularly preferred for orally disintegrating tablets. Quick dissolve delivery systems typically comprise polyols, for instance spray-dried polyols in addition to other excipients. An example of a suitable quick dissolve delivery system is Pharmaburst from SPI Pharma. This is a co-processed excipient system comprising mannitol, sorbitol, crospovidone and silica (colloidal hydrated). When this is used, it is possible to have an orally disintegrating tablet comprising no disintegrant. Typically, the quick dissolve delivery system comprises 40-80% by weight of the orally disintegrating tablet, most preferably 70-80% by weight.

The disintegrant of the orally disintegrating tablet may be selected from the group consisting of crosslinked sodium carboxymethylcellulose, crosslinked polyvinylpyrrolidone, crosslinked carboxymethyl starch, different types of starch and microcrystalline cellulose, magnesium aluminium silicate and any combinations thereof.

The lubricant/glidant of the orally disintegrating tablet may be selected from the group consisting of magnesium, calcium and zinc stearate, calcium behenate, sodium stearyl fumarate, talc, magnesium trisilicate, stearic acid, palmitic acid, carnauba wax, silicon dioxide and any combinations thereof.

Formation of the coated micropellets into tablets involves a choice of suitable excipients and compression parameters. It is important that the micropellets are robust and that they and their coating deform without fracture. When the tablet is formed an inert compression diluent is preferably selected as one of the excipients. In this context, the diluent should, in addition to all normal tableting considerations, have minimal segregation propensity, cushion the micropellets during compression so as to minimise damage, disintegrate rapidly to release intact micropellets and have minimal effect on drug release kinetics.

Preferably, in the method according to the second aspect of this invention, the extrusion-spheronisation technique is used. To produce micropellets having a diameter of less than 500 µm, a screen size of less than 500 µm is used in the process. More preferably, micropellets having an average diameter of less than 350 µm are produced, and a screen size of less than 350 µm is used. Extrusion-spheronisation is a two step process wherein the micropellet ingredients are mixed together to form a wet powder mass and this is extruded, followed by a spheronisation process which produces the spherical pellets, followed by drying.

For a more detailed description of the spheronization process, reference is made to "A New Technique for the Production of Spherical Particles" by A.D.Reynolds in Manufacturing Chemist, Aerosol News, V41, (Jun), p40-43, 1970 and "Pelletization Techniques" by James Swarbick and J.C. Boylan in Encyclopedia of Pharmaceutical Technology, Vol. II. Marcel Dekker, 1995: 369-394.

Other techniques may be used to produce the substantially spherical micropellets, such as high shear granulation or rotogranulation.

Preferably, the micropellets produced in the method are substantially spherical and have a smooth surface.

The micropellets may be coated by spraying with the coating composition. A fluidised bed apparatus is suitably used for coating the micropellets in step (ii) with the coating composition. Other techniques known in the art, such as coacervation, may also be utilised.

For the production of orally disintegrating tablets, typically a mixture of the coated micropellets and the one or more excipients is prepared, and then homogenized in a mixer. The mixture is then typically subjected to a compression force sufficient to render the resulting tablet sufficiently hard for it to be handled and packaged industrially and handled by the patient.

The following Examples illustrate the invention in more detail.

### Example 1

### Composition of Ketoprofen ODT

### 1.1 Ketoprofen Micropellets

**Table 2. Composition of Ketoprofen micropellets**

| **Ingredient** | **Mass (mg)** |
|---|---|
| Ketoprofen | 100.00 |
| MCC (microcrystalline cellulose) | 12.50 |
| Polysorbate 80 | 2.00 |
| Carrageenan | 6.25 |
| Lactose | 6.25 |
| Aqua | 31.72 |

### Preparation of Ketoprofen Micropellets

Micropellets are prepared by extrusion and spheronisation.

Polysorbate is dissolved in one part of purified water.

Ketoprofen, microcrystalline cellulose, carrageenan and lactose are homogeneously mixed and the agglomeration liquid is added to the mixture of dry powder in a planetary mixer (PRS, Erweka, Heusenstamm, Germany).

The wet powder mass is extruded at 60 rpm in an axial screen extruder (Pharmex 35T, Wysstec, Pery, Switzerland) equipped with a 0.4 mm thick screen with 0.35 mm diameter circular openings. The housing of extruder is cooled with water. The extrudate is spheronised on a radial plate spheroniser (SPHEROMAT 250T, Wysstec, Pery, Switzerland). Extrudates are spheronised for 0.5 min at 1500 rpm and additionally 1.5 min at 1200 rpm.

Pellets are dried in a drier with forced convection for 2 hours at 40°C.

Sieve analysis is performed to determine the size of micropellets (Table 3):

**Table 3.**

| **Sieve (mm)** | **% of the total** |
|---|---|
| 0.8 | 1.16 |
| 0.63 | 1.16 |
| 0.5 | 1.37 |
| 0.4 | 3.80 |
| 0.315 | 68.88 |
| 0.250 | 19.00 |
| Bottom | 4.62 |

### 1.2 Micropellet Coating

**Table 4. Composition of a coating**

| **Ingredient** | **Mass (mg)** |
|---|---|
| Pharmacoat (HPMC - Hydroxy propyl methyl cellulose) | 19.05 |
| PEG | 3.81 |
| Talc | 4.35 |
| Aroma | 1.00 |
| Aqua | 348.0 |

### 1. Preparation of a Coating and Coating of Micropellets

Polymer coating dispersion is prepared with the aid of propeller stirrer. Pharmacoat is added steadily and quickly to the vortex, avoiding powder flotation on the liquid surface. The stirrer speed is increased in order to maintain the vortex. Special grade of talc is used with very small particles with diameter 1.1 µm in order to effectively coat the micropellets. It is homogenised separately with the use of Ultra Turrax. Polymer dispersion and glidant suspension are combined. Finally, plasticiser and aroma are added.

During spraying it is important to avoid sedimentation.

Micropellets are coated in a fluidised bed apparatus (bottom spray i.e. Glatt GPCG 1, Wurster insert or tangential spray i.e. Hüttlin HKC 5). Coating thickness is measured with SEM analysis and is found to be 7-10 microns.

Using SEM, coated micropellet diameter is found to be 320 µm.

### 1.3 Ketoprofen ODT

**Table 5. Composition of ODT**

| **Ingredient** | **Mass (mg)** |
|---|---|
| Coated micropellets | 155.21 |
| Quick dissolve delivery system for tablets | 219.99 |
| Aroma | 8.80 |
| Sodium stearyl Fumarate | 16.00 |
| **TOTAL** | **400.00 mg** |

### 2. Preparation of Ketoprofen ODT

Triturate of aroma with a part of filler is prepared and homogeneously mixed with the rest of filler. Coated micropellets and filler are homogeneously mixed. The coated micropellets comprise 127 mg of micropellets and 28.21 mg of coating composition. Finally, sodium stearyl fumarate is added and homogeneously mixed and compressed into tablets, mass 400 mg, in a room with controlled low relative air humidity.

### Example 2

### 3. Composition of Ketoprofen ODT

### 2.1 Ketoprofen Micropellets

**Table 6. Composition of Ketoprofen Micropellets**

| **Ingredient** | **Mass (mg)** |
|---|---|
| Ketoprofen | 100.00 |
| MCC (microcrystalline cellulose) | 12.50 |
| Polysorbate 80 | 2.00 |
| Carrageenan | 6.25 |
| Lactose | 6.25 |
| Aqua | 31.72 |

### 4. Preparation of Ketoprofen Micropellets

Micropellets are prepared by extrusion and spheronisation.

Polysorbate is dissolved in one part of purified water.

Ketoprofen, microcrystalline cellulose, carrageenan and lactose are homogeneously mixed and the agglomeration liquid is added to the mixture of dry powder in a planetary mixer (PRS, Erweka, Heusenstamm, Germany).

The wet powder mass is extruded at 60 rpm in an axial screen extruder (Pharmex 35T, Wysstec, pery, Switzerland) equipped with a 0.4 mm thick screen with 0.35 mm diameter circular openings. The housing of extruder is cooled with water. The extrudate is spheronised on a radial plate spheroniser (SPHEROMAT 250T, Wysstec, Pery, Germany). Extrudates are spheronised for 0.5 min at 1500 rpm and additionally 1.5 min at 1200 rpm.

Pellets are dried in a drier with forced convection for 2 hours at 40°C.

### 2.2 Micropellet Coating

**Table 7. Composition of a Coating**

| **Ingredient** | **Mass (mg)** |
|---|---|
| Eudragit E PO | 25.00 |
| Sodium lauryl sulfate | 2.50 |
| Stearic acid | 3.75 |
| Glycerol monostearate | 1.25 |
| Polysorbate 80 | 0.50 |
| Aroma | 1.00 |
| Aqua | 216.00 |

### 5. Preparation of a Coating and Coating of Micropellets

Polymer coating dispersion is prepared with the aid of Ultra Turrax (high shear forces homogeniser).

Sodium lauryl sulfate, stearic acid, Eudragit E PO are added sequentially to the water and homogenised.

Glidant emulsion is prepared separately. Polysorbate 80 and glycerol monostearate are homogenised in the heated water using a homogeniser (Ultra Turrax/high shear mixer). The glycerol monostearate emulsion is mixed with a part of remaining amount of water and then cooled down to room temperature while stirring with a conventional stirrer.

The glidant emulsion is slowly poured into the Eudragit dispersion while gently stirring. During spraying it is important to avoid sedimentation.

Micropellets are coated in a fluidised bed apparatus (bottom spray i.e. Glatt GPCG 1, Wurster insert of tangential spray, i.e. Hüttlin HKC 5). Coating thickness is measured with SEM analysis and is found to be approximately 20 microns.

Using SEM, coated micropellet diameter is found to be 320 µm.

### 2.3 Ketoprofen

**Table 8. Composition of ODT**

| **Ingredient** | **Mass (mg)** |
|---|---|
| Coated micropellets | 161.00 |
| Quick dissolve delivery system for tablets | 214.20 |
| Aroma | 8.80 |
| Sodium stearyl Fumarate | 16.00 |
| **TOTAL** | **400.00 mg** |

### 6. Preparation of Ketoprofen ODT

Triturate of aroma with a part of filler is prepared and homogeneously mixed with the rest of filler. Coated micropellets and filler are homogeneously mixed. The coated micropellets comprise 127 mg of micropellets and 34 mg of coating composition. Finally, sodium stearyl fumarate is added and homogeneously mixed and compressed into tablets, mass 400 mg, in a room with controlled low relative air humidity.

## Claims

1. An orally disintegrating tablet comprising micropellets of which at least 50% by weight have a diameter less than 500 µm and one or more excipients; wherein the micropellets comprise a pharmaceutically active ingredient and one or more auxiliary ingredients and are coated with a coating composition.

2. An orally disintegrating tablet according to claim 1, wherein the one or more auxiliary ingredients include microcrystalline cellulose and/or carrageenan.

3. An orally disintegrating tablet according to claim 1 or 2, wherein the one or more auxiliary ingredients include lactose.

4. An orally disintegrating tablet according to any of the preceding claims, wherein the one or more auxiliary ingredients include one or more disintegrants and/or lubricants and/or wetting agents or surfactants.

5. An orally disintegrating tablet according to any one of the preceding claims, wherein the coating composition comprises a polymethacrylate or a cellulose-based polymer.

6. An orally disintegrating tablet according to claim 5, wherein the coating composition comprises hydroxypropyl methyl cellulose, ethyl cellulose, hydroxyethyl cellulose, carboxymethylcellulose sodium, polyvinyl alcohol, polyvinyl pyrrolidone or any combinations thereof.

7. An orally disintegrating tablet according to claim 5, wherein the coating composition comprises an aminoalkyl methacrylate copolymer.

8. An orally disintegrating tablet according to any of claims 5-7, wherein the coating composition additionally comprises polyethylene glycol, lactose, HPMC, HPC and/or microcrystalline cellulose.

9. An orally disintegrating tablet according to any of claims 5-8, wherein the coating composition additionally comprises one or more plasticizers, glidants, pigments and/or solubilising agents.

10. An orally disintegrating tablet according to any preceding claim, wherein the one or more excipients comprise a filler and/or a disintegrant and/or a lubricant/glidant.

11. An orally disintegrating tablet according to claim 10, wherein the filler is selected from the group consisting of microcrystalline cellulose (MCC), modified forms of microcrystalline cellulose, lactose, sugars, starch or a modified starch, mannitol, polyols, preferably sorbitol, dextrin, dextran and maltodextrin, calcium carbonate, calcium phosphate and/or hydrogen phosphate, sulphate or any combinations thereof.

12. An orally disintegrating tablet according to claim 10 or 11, wherein the disintegrant is selected from the group consisting of crosslinked sodium carboxymethylcellulose, crosslinked polyvinylpyrrolidone, crosslinked carboxymethyl starch, different types of starch and microcrystalline cellulose, magnesium aluminium silicate or any combinations thereof.

13. An orally disintegrating tablet according to any of claims 10-12, wherein the lubricant/glidant is selected from the group consisting of magnesium, calcium and zinc stearate, calcium behenate, sodium stearyl fumarate, magnesium trisilicate, stearic acid, palmitic acid, carnauba wax, silicon dioxide or any combinations thereof.

14. An orally disintegrating tablet according to any of the preceding claims, wherein at least 50% by weight of the micropellets have a diameter of less than 350 µm.

15. An orally disintegrating tablet according to any of the preceding claims, wherein the pharmaceutically active ingredient is selected from diclofenac, ketoprofen, ibuprofen, aspirin, paracetamol, melatonin and pharmaceutically acceptable salts thereof.

16. An orally disintegrating tablet according to any of the preceding claims, wherein the pharmaceutically active ingredient comprises 50-90% wt of the micropellets.

17. An orally disintegrating tablet according to claim 16, wherein the pharmaceutically active ingredient comprises at least 70% wt of the micropellets.

18. Method of making an orally disintegrating tablet according to any of claims 1-17, comprising
(i) forming micropellets of which at least 50% by weight have a diameter of less than 500 µm from a pharmaceutically active ingredient and one or more auxiliary ingredients;
(ii) coating the micropellets formed in step (i) with a coating composition; and
(iii) compressing the coated micropellets produced in step (ii), together with one or more excipients to form a tablet.

19. Method according to claim 18, wherein the micropellets in step (i) are formed by extrusion-spheronisation using an extrusion screen having a screen size of less than 500 µm.

20. Method according to claim 19, wherein the screen size is less than 350 µm.

21. Method according to any one of claims 18-20, wherein in step (ii) the micropellets are coated in a fluidised bed apparatus.

22. Use of an orally disintegrating tablet according to any of claims 1-17 to administer the pharmaceutically active ingredient to a patient.
